# EUROPEAN PATENT SPECIFICATION

(11) **EP 2 170 331 B1**
(45) Date of publication and mention of the grant of the patent: **03.12.2014**
(21) Application number: 08774299.5
(22) Date of filing: 25.06.2008
(51) Int. Cl.: A61K 36/15, A61P 39/06, A61K 31/352, A61K 31/353, A61K 31/355, A61K 31/455

(54) **SYNERGISTIC COMBINATION OF PROANTHOCYANIDINS, GAMMA-TOCOTRIENOL AND NIACIN**
SYNERGISTISCHE KOMBINATION VON PROANTHOYANIDINEN, GAMMA-TOCOTRIENOL UND NIACIN
COMBINAISON SYNERGIQUE DE PROANTHOCYANIDINES, DE GAMMA-TOCOTRIÉNOL ET DE NIACINE

(30) Priority: 29.06.2007 EP 07111429
(43) Date of publication of application: 07.04.2010
(73) Proprietor: LABORATOIRES DR NG PAYOT, 75008 Paris (FR)
(72) Inventor: BALAGUER SANCHO, Francisco, E-08024 Barcelona (ES); PANYELLA COSTA, David, E-08912 Badalona (Barcelona) (ES); GINESTAR GONZÁLEZ, José, E-08320 El Masnou (Barcelona) (ES); RECASENS GRACIA, Mar, 08005 Barcelona (ES)
(74) Representative: ZBM Patents - Zea, Barlocci & Markvardsen
(86) International application number: PCT/EP2008/058109
(87) International publication number: WO 2009/003899

(56) References cited:
- EP-A1- 1 609 460
- WO-A-94/13265
- WO-A-2005/115378
- US-A- 5 972 999
- US-A1- 2004 191 202
- MAULIK GAUTAM ET AL: "Oxygen free radical scavenging properties of proanthocyanidins" FREE RADICAL BIOLOGY AND MEDICINE, vol. 27, no. SUPPL. 1, 1999, page S39, XP009104483 & 6TH ANNUAL MEETING OF THE OXYGEN SOCIETY; NEW ORLEANS, LOUISIANA, USA; NOVEMBER 18-22, 1999 ISSN: 0891-5849
- PACKER L ET AL: "ANTIOXIDANT ACTIVITY AND BIOLOGIC PROPERTIES OF A PROCYANIDIN-RICH EXTRACT FROM PINE (PINUS MARITIMA) BARK, PYCNOGENOL" FREE RADICAL BIOLOGY AND MEDICINE, ELSEVIER SCIENCE, XX, vol. 27, no. 5/06, 1 January 1999 (1999-01-01), pages 704-724, XP001120382 ISSN: 0891-5849
- NEWAZ M A ET AL: "EFFECT OF GAMMA-TOCOTRIENOL ON BLOOD PRESSURE, LIPID PEROXIDATION AND TOTAL ANTIOXIDANT STATUS IN SPONTANEOUSLY HYPERTENSIVE RATS (SHR)" 1 January 1999 (1999-01-01), CLINICAL AND EXPERIMENTAL HYPERTENSION, MARCEL DEKKER, NEW YORK, NY, US, PAGE(S) 1297 - 1313 , XP002942420 ISSN: 1064-1963 the whole document
- NEWAZ M A ET AL: "NITRIC OXIDE SYNTHASE ACTIVITY IN BLOOD VESSELS OF SPONTANEOUSLY HYPERTENSIVE RATS: ANTIOXIDANT PROTECTION BY GAMMA-TOCOTRIENOL" JOURNAL OF PHYSIOLOGY AND PHARMACOLOGY, POLISH PHYSIOLOGICAL SOCIETY, KRAKHAW, PL, vol. 54, no. 3, 1 January 2003 (2003-01-01), pages 319-327, XP009058416 ISSN: 0867-5910
- PREUSS HARRY G ET AL: "Protective effects of a novel niacin-bound chromium complex and a grape seed proanthocyanidin extract on advancing age and various aspects of syndrome X" ANNALS OF THE NEW YORK ACADEMY OF SCIENCES. ALCOHOL AND WINE IN HEALTH AND DISEASE NEW YORK ACADEMY OF SCIENCES {A}, 2 EAST 63RD STREET, NEW YORK, NY, 10021, USA SERIES : ANNALS OF THE NEW YORK ACADEMY OF SCIENCES (ISSN 0077-8923), 2002, pages 250-259, XP009104468 & CONFERENCE ON ALCOHOL AND WINE IN HEALTH AND DISEASE; PALO ALTO, CA, USA; APRIL 26-29, 2001 ISSN: 1-57331-376-9 1-57331-377-7

## Description

The present invention relates to compositions comprising a combination of a source of proanthocyanidins which is a pine bark extract from Pinus pinaster gamma-tocotrienol and niacin. Due to synergistic effects, the protective effects of these synergistic combinations are useful in treating or preventing various cosmetic conditions and dermatological disorders.

### BACKGROUND OF THE INVENTION

One of the actual strategies for protecting biomolecules is the support of the endogenous antioxidant system. Topical administration of nonenzymatic antioxidants has found to be very effective in, for example, photoprotection.

It has been found that single components of the antioxidant system are successful against damage caused by reactive oxygen species (ROS). However the right balance between the various components of the antioxidant network is thought to be crucial.

The most promising results in preventing damaged caused by ROS were obtained in studies combining several antioxidants resulting in synergism of the protective effects. The way to optimize the antioxidant network remains elusive and is an area of intense research.

Radical scavenger recyclers capable of recycling tocotrienols have been described. These radical scavenger recyclers consist of alpha lipoic acid, ascorbic acid, derivatives of such acids and their mixtures.

Proanthocyanidins designate a group of flavanoids that includes the subgroups procyanidins, prodelphinidins and propelargonidins. Proanthocyanidins are homogeneous or heterogeneous polymers consisting of the monomer units catechin or epicatechin, which are connected either by 4-8 or 4-6 linkages, to the effect that a great number of isomer proanthocyanidins exist. Typically, the proanthocyanidins oligomers have a chain length of 2-12 monomer units. Proanthocyanidins may be synthesized or extracted from a plant material.

Proanthocyanidins are known to have antioxidant activity as free radical scavenger. Furthermore, proanthocyanidins are known to have beneficial properties in the management of cardiovascular health as circulation enhancer, cholesterol reduction, and blood pressure control; in attention deficit disorders; in dermatology as sun protection, skin elasticity, wrinkle reduction, anti-aging, wound healing, skin nutrition and pigmentation problems; in diabetes in the blood glucose management, diabetic syndrome, diabetic retinopathy and leg ulcers and microangiopathy; in fertility to improve sperm quality; as anti-inflammatory agent; in osteoarthritis relief, menstrual pain relief, endometriosis, gingival bleeding, stop plaque formation, better healing, energy enhancer, muscle recovery, pain and cramp reduction, enhancer of production of vascular nitric oxide, thrombosis, edema, venous insufficiency, among others.

Proanthocyanidins are found in high concentrations from such sources as: cramberries, grape skins, grape seeds, pine bark, lemon tree bark, ginkgo leaves, peanuts, and cocoa beans, tamarind, tomato, peanut, almond, apple, blueberry, tea leaves and hazel nut tree leaves. The two most common and richest known sources are grape seed extract and pine bark extract.

A well-known product containing proanthocyanidins, which is available in trade as a preparation of a food supplement under the name Pycnogenol®, is a registered trademark belonging to Horphag Research, Ltd. The Pycnogenol® food supplement contains approximately 70-80% of proanthocyanidins and is a complex mixture of phenolic substances. Besides proanthocyanidins and its monomeric unit catechin, Pycnogenol® contains taxifolin and a wide range of phenolic acids, e.g. free acids like p-hydroxybenzoic acid, protocatechic acid, vanillic acid, caffeic acid and ferulic acid as well as its glucosides and glucose esters (Rohdewald P. Int J Clin Pharmacol Therap, 2002 40(4):158-168).

Topical application of proanthocyanidin-containing combinations to treat skin disorders are described in various documents.

US 5470874 describes the use of topically applied mixtures of vitamin C and proanthocyanidins for human skin care, achieving a synergistic effect as for the scavenging of free radicals.

US 5972999 protects the use of a combination consisting of a sugar compound, an antioxidant an amino acid and a transition metal to repair skin for the prevention and treatment of wrinkles and other skin disorders.

Tocotrienols comprise one of the two groups of molecules belonging to the vitamin E family, the other group being the tocopherols. Tocotrienols and tocopherols are sometimes collectively called tocols. Just as there are four natural tocopherols, alpha-, beta-, gamma- and delta-tocopherol, there are also four natural tocotrienols, alpha-, beta-, gamma- and delta-tocotrienol. The tocotrienols differ from the tocopherols in the chemical nature of the side chain or tail. Tocopherols have a saturated phytyl side chain, whereas tocotrienols have an unsaturated isoprenoid or famesyl side chain possessing three double bonds.

The major source of tocotrienols are plant oils, and the richest sources are palm oil, rice bran oil, palm kernel oil and coconut oil. Tocotrienols are also found in such cereal grains as oat, barley and rye. Vegetable oils, such as those from canola, cottonseed, olive, peanut, safflower, soybean and sunflower, contain little to no tocotrienols. However, those oils do contain tocopherols. Corn oil has small amounts of tocotrienols.

All of the natural tocotrienols are fat-soluble, water-insoluble oils. The tocotrienols, as well as the tocopherols, possess chain-breaking, peroxyl radical scavenging activities. In contrast to tocopherols, tocotrienols inhibit the rate- limiting enzyme of the cholesterol biosynthetic pathway beta-hydroxy-beta-methylglutaryl-coenzyme A (HMG-CoA) reductase.

The four natural tocotrienols are characterized by the number of methyl groups and their position in the chromanol ring. Alpha-tocotrienol has three methyl groups located on positions 5, 7 and 8 of the chromanol ring; beta-tocotrienol has two methyl groups located on positions 5 and 8 of the ring; gamma-tocotrienol has two methyl groups located on positions 7 and 8 of the ring and delta-tocotrienol has one methyl group located on position 8 of the ring.

While tocopherols have three chiral centers, tocotrienols have only one chiral center. Therefore, each tocotrienol has two stereoisomeric possibilities in comparison with eight such possibilities for each of the tocopherols. The natural tocotrienols exist as d-stereoisomers: d-alpha-tocotrienol, d-beta-tocotrienol, d-gamma-tocotrienol and d-delta-tocotrienol. The chiral center in the tocotrienol structure is at the point where the isoprenoid side chain bonds to the chromanol ring, the 2 position of the ring. The natural tocotrienols are E, E in reference to the geometric configuration of the double bonds of the side chain.

D-Alpha-tocotrienol is also known as 2R, 3'E, 7'E-alpha tocotrienol and is abbreviated alpha-T₃; d-beta-tocotrienol is known as 2R, 3'E, 7'E-beta-tocotrienol and abbreviated beta-T₃; d-gamma-tocotrienol is known as 2R, 3'E, 7'E-gamma-tocotrienol and abbreviated gamma-T₃; delta-tocotrienol is known as 2R, 3'E, 7'E-delta-tocotrienol and abbreviated delta-T₃. Tocotrienols are presently available in nutritional supplement form as mixed tocotrienols. Typically, the gamma form is the most abundant one in the mixture.

Tocotrienols have antioxidant activity. They may also have hypocholesterolemic, anti-atherogenic, antithrombotic, anticarcinogenic and immunomodulatory actions.

Some studies have shown tocotrienols to be more effective inhibitors of both lipid peroxidation and protein oxidation than alpha-tocopherol. In these same studies, the order of antioxidant potency was gamma-tocotrienol followed by the alpha and delta tocotrienol homologues; in this respect a controversy exists provided that in other studies the order can be different. Gamma-tocopherol is a peroxynitrite scavenger, and gamma-tocotrienol may be, as well. The possible greater antioxidant activity of the tocotrienols, compared with alpha-tocopherol, may be accounted for by a more efficient interaction of the chromanol ring with reactive oxygen species, a higher recycling efficiency of the chromanoxyl radicals of the tocotrienols and a more uniform distribution of the tocotrienols in cellular membranes. In any case, alpha-tocopherol is the dominant tocol in human plasma and tissue, and, therefore, would be expected to be the dominant antioxidant form, as well.

Some research suggests tocotrienols are more potent in their antioxidation and anti-cancer effect than the common forms of tocopherol due to significant differences in chemical structure. The unsaturated side-chain in tocotrienols makes them penetrate tissues with saturated fatty layers more efficiently, making them potentially more useful for cosmetic and dermatological products.

Tocotrienols inhibit the rate-limiting enzyme of the cholesterol biosynthetic pathway, beta-hydroxy-beta-methlyglutaryl-coenzyme A (HMG-CoA) reductase. Tocopherols do not have this activity. Tocotrienols' possible antithrombotic effect may be due to tocotrienols' (especially gamma-tocotrienol's) inhibition of thromboxane B₂ synthesis, as well as their suppression of plasma levels of platelet factor 4.

Although all the isomers of the tocotrienol family are important antioxidants due to the ease of donating a hydrogen atom from the hydroxyl group on the chromanol ring to reduce free radicals, each of them has its own biological activity.

There are some data suggesting that tocotrienols may have greater hypolipidemic effects than the tocopherols. The tocotrienols show some promise in inhibiting breast cancer and some other malignancies. Tocotrienols were reported to promote healing of ethanol-induced gastric lesions.

Antioxidant combinations consisting of proanthocyanidins and tocotrienols have been disclosed in the prior art, for example EP 1331858 that discloses the use of synergistic antioxidant combinations of delta tocols and polyphenols. Such antioxidants agents containing polyphenols may include proanthocyanidins of maritime pine bark. These compositions are based on a source of delta-tocol comprising greater than 2% by weight delta-tocol.

Similarly EP 141549 discloses a combination of polyphenols and tocols consisting of a source of non-alpha-tocol comprising from 2% to 95% by weight non alpha-tocol.

Skin lightening compositions containing a flavonoid, vitamin C, vitamin -E and niacin have been described in WO 2005/094770.

NZ 526350 describes anti-aging compositions comprising an amino acid, vitamin B complex, metal salt and an amino alcohol.

Complex antioxidant compositions have been described, for example US 20050048008 discloses a system based on an extra-cellular antioxidant (or free-radical neutralizing composition) and an intra-cellular antioxidant (or free-radical neutralizing composition) and anti-inflammatory composition and a collagen or fibring boosting composition in a topical delivery system. The extra-cellular antioxidant composition comprises quaternary ion pairs of ionic antioxidants.

US 20040146539 describes mixtures of antioxidant compositions in complex topical nutraceutical compositions with selective body slimming and tone firming antiaging benefits.

WO 2000/57876 describes antioxidant formulations and methods for using them comprising tocotrienols and radical scanvenger recyclers capable of recycling them. The radical scavenger recycler is selected from the group consisting of alpha-lipoic acid, alpha-lipoic acid salts, derivatives of alpha-lipoic acid, ascorbic acid, ascorbic acid salts, derivatives of ascorbic acid and mixtures of these compounds. In this case the use of radical scavenger recyclers (glutathione, vitamin C or alpha lipoic acid) is required.

Thus, despite the teachings of these prior art, there is still a need for new cosmetic, pharmaceutical and/or dietary compositions useful against damage caused by reactive oxygen species (ROS) with an improved activity.

### SUMMARY OF THE INVENTION

Inventors have examined several described antioxidant compositions and it has been found that mixtures of proanthocyanidins, gamma tocotrienol and niacin present a synergistic effect in preventing cell damage against oxidative stress, as well as a synergistic cell renewal effect.

The antioxidants alone did not show any remarkable protective effect in preventing the oxidative stress mediated by reactive oxygen species in an *in vitro* assay. As previously mentioned, synergistic systems containing flavonoids and delta tocols have been described. Nevertheless, the compositions of the present invention contain gamma tocotrienol, which did not show any synergistic effect with the proanthocyanidin unless niacin is present. Nevertheless, the composition containing the three components (proanthocyanidins, gamma-tocotrienol and niacin) shows an improved synergistic effect versus a binary composition comprising proanthocyanidins and gamma-tocotrienol.

Accordingly, a first aspect of thg invention relates to a composition comprising a source of proanthocyanidins which is a pine bark extract from Pinus pinaster, gamma-tocotrienol and niacin.

In a further aspect of the invention, it is provided a pharmaceutical or cosmetical composition or a dietary supplement comprising a therapeutical, cosmetical or dietary effective amount of the composition of the invention and pharmaceutically, cosmetically, or dietary acceptable excipients or carriers.

In yet another aspect of the invention there is provided a composition according to the invention for use as a medicament.

In a further aspect of the invention, it is provided the use of a composition according to the present invention, in the manufacture of a medicament for the prevention and/or treatment of a disease condition or corporal damage associated with the generation of free radicals.

In a further aspect of the invention, it is provided the use of a composition according to the present invention, for the cosmetic or dietary prevention and/or treatment of a disease condition or corporal damage associated with the generation of free radicals.

### BRIEF DESCRIPTION OF THE DRAWINGS

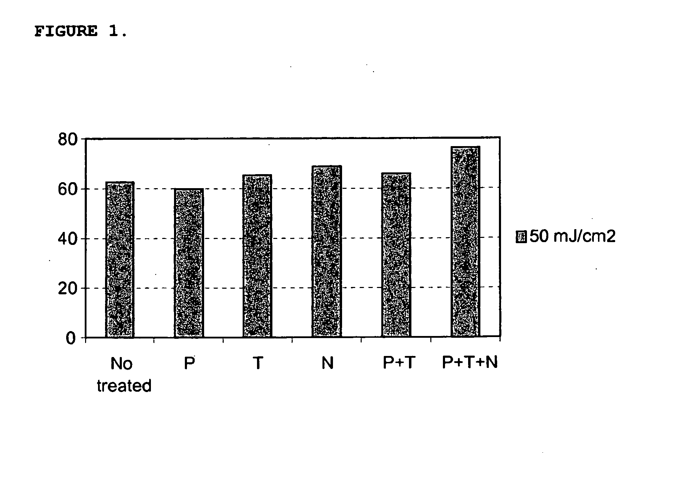

FIG. 1. Shows the synergistic effect of the composition of the invention in preventing cell damage against UV stress. Synergistic effect is showed as % of viability of treatments 24 hours after UVB irradiation (50 mJ/cm²) versus no treated and irradiated.
   P= pycnogenol^{®} 0.05 mg/ml
   T= gamma-tocotrienol 0.01 mg/ml
   N = niacinamide 0.025 mg/ml
   P+T= pycnogenol^{®} 0.05 mg/ml + gamma-tocotrienol 0.01 mg/ml
   P+T+N = pycnogenol^{®} 0.05 mg/ml + gamma-tocotrienol 0.01 mg/ml + niacinamide 0.025 mg/ml
   * p<0.05 (ANOVA) versus no treated and irradiated.

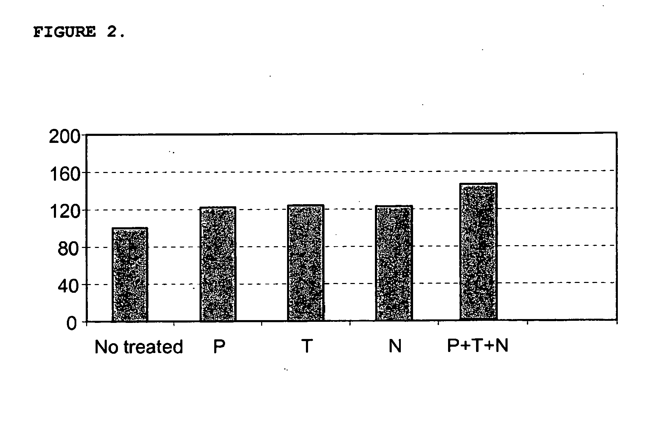

FIG. 2. Shows the synergistic effect of the composition of the invention in a proliferation study. Synergistic effect is showed as % of viability of treatments versus no treated.
   P= pycnogenol^{®} 0.05 mg/ml
   T= gamma-tocotrienol 0.01 mg/ml
   N = niacinamide 0.025 mg/ml
   P+T= pycnogenol^{®} 0.05 mg/ml + gamma-tocotrienol 0.01 mg/ml
   P+T+N = pycnogenol^{®} 0.05 mg/ml + gamma-tocotrienol 0.01 mg/ml + niacinamide 0.025 mg/ml
   * p<0.05 (Student's t-test).

### DETAILED DESCRIPTION OF THE INVENTION:

The proanthocyanidins of this disclosure are limited to those derived from a source of proanthocyanidins which is a French maritime pine bark extract (Pycnogenol®), obtained from *Pinus pinaster,* syn. *Pinus maritima.*

In the context of the present invention, the term Pycnogenol^{®} refers to an extract of the bark of the maritime pine, *Pinus maritime,* which contains proanthocyanidins.

According to an embodiment of the invention, the Pycnogenol^{®} extract is produced according to U.S. Patent 5,698,360.

The extract used according to the invention may be prepared essentially by extracting maritime pine bark using water or an organic solvent. When water is used, it is preferably to employ warm water or hot water. In order to increase the extraction efficiency, it is preferable to add a salt such as sodium chloride to the water. As the organic solvent that can be employed for extraction, an organic solvent that is acceptable for production of dietary supplements, cosmetic, dermatologic or pharmaceutical compositions can be employed. Examples of such solvents include methanol, ethanol, 1-propanol, 2-propanol, 1-butanol, acetone, hexane, cyclohexane, propylene glycol, methyl ethyl ketone, glycerine, methyl acetate, ethyl acetate, diethyl ether, dichloromethane, and edible oils or fats. These water and the organic solvents may be used alone or in combination.

In a particular extraction procedure, the extraction is carried out by using boiled water, saturating the filtered extract with sodium chloride or, alternatively, adding ammonium sulphate to 20% w/v, separating the precipitate formed, repeatedly extracting the supernatant with 1/10 volumes of ethyl acetate, drying the collected ethyl acetate extracts, concentrating the dried extract, pouring it into 3 volumes of chloroform with stirring and collecting the precipitate which may be purified by repeating the dissolution in ethyl acetate and precipitation with chloroform.

The method for extracting proanthocyanidins from the pine bark is not particularly limited, then other extraction methods leading to the same composition of the extract may also be used to prepare this extract.

In the context of the present invention, niacin is the generic name for a group of compounds which exhibit niacin activity, and includes niacinamide and nicotinic acid. Preferably, niacin is provided as niacinamide.

The cosmetic, dermatological or pharmaceutical formulations of the invention, comprises the source of proanthocyanidins, the source of gamma-tocotrienol and niacin, and optionally one or more cosmetically, dermatologically and/or pharmaceutically acceptable excipients.

Contrary to the observed in EP1331858, wherein delta tocols are better synergistic partners than any of the other tocol isomers, the inventors have found that gamma-tocotrienol in combination with a source of proanthocyanidins and niacin shows an improved synergistic effect.

In one embodiment of the invention, the total weight content of proanthocyanidins, gamma-tocotrienol and niacin in the composition of the invention is between 0.01% and 2% for each component. Preferably between 0.5% and 1%.

According to an embodiment of the invention, the composition is substantially free of delta-tocols (delta-tocopherol and delta-tocotrienol), meaning that less than 5%, preferably less than 2% by weight of the total content of the composition is delta-tocol. Most preferably, the source of gamma-tocotrienol is a Tocotrienol Rich Fraction (TRF) with an amount of delta-tocols below 2% by weight

The compositions according to the invention is preferably administered orally or applied topically to the areas to be treated. The administration or application may be performed, for example, daily, for a duration of several weeks, and the treatment may be renewed periodically, according to the individual to be treated.

The subject compositions may also be formulated for administration by other routes, including without limitation, buccal, sublingual, rectal, parenteral, inhalational, including intranasal, injectable, including subcutaneous, intravenous, intramuscular, etc., including transdermal, etc

The cosmetic, dermatological or pharmaceutical formulation can be adapted for application thereof topically, particularly onto the skin and/or mucous membranes such as buccal cavity.

In a preferred embodiment of any of the cosmetic, dermatological or pharmaceutical formulation according to the invention the formulation is in the form, without limitation, of a day cream, eye contour cream, body milk, mouthwash, pencils, cover sticks, acne sticks, lipsticks, make-ups, foundations, face powder, blusher, eyeshadows, eyeliners, peel creams, hair waxes, hair styling compositions, styling fluids, hair foams, hair gels, hair sprays, mousse, hair oil, end fluids, hair treatments, night creams, care creams, nutrient creams, perfume creams, body lotions, ointments, lipcare compositions, sunscreen compositions, deodorants, antiperspirants, colored gels in the form of pencils, such as, for example, multiphase pencils, sticks, pastes, powder, creams, cream foams, lotions, self-foaming, foam-like, after-foaming or foamable emulsions, gels, roll-on preparations, foams, depilatories, liquid, solution, suspension, emulsion, multiple emulsion, tablet, multi-layer tablet, bi-layer tablet, capsule, gelatin capsule, caplet, lozenge, chewable lozenge, bead, powder, granules, dispersible granules, cachets, douche, suppository, aerosol inhalant, patches, adhesive patches, cleansing cushions, hydrogels, wipes, face masks, particle inhalant, implant, depot implant, ingestible, injectable, or infusion.

The cosmetic, dermatologic or pharmaceutical formulation or dietary supplement according to the invention may be used for the administration or application to mammals, preferably for humans including infants, children and adult.

A "dietary supplement" shall mean a product that is intended to supplement the human diet and is typically provided in the form of a pill, powder, capsule, tablet or like formulation. The terms "dietary" or "nutritional" can be used interchangeably.

The synergistic compositions of the invention can readily be incorporated into nutritional formulations, typically nutraceuticals or functional food of beverage products. In general, food and beverage products of the invention will comprise nutritional quantities of one or more of carbohydrates, protein, fibre and fat. The edible products of the invention optionally comprise conventional food additives, such as any of emulsifiers, stabilizers, sweeteners, flavourings, colouring agents, preservatives, chelating agents, osmotic agents, acidulants, thickeners, texturisers, and so on.

If desired, other active ingredients may be incorporated into the formulations of the invention.

In selecting the excipients, adjuvants, etc., a skilled in the art will ensure that they do not affect the activity of the composition in accordance with the invention.

The composition of the cosmetic or pharmaceutical composition or dietary supplement may vary depending on the use. Conventional pharmaceutical, cosmetical and/or dietary excipients or carriers as well as processing techniques and the like are set forth in e.g. Remington's Pharmaceutical Sciences, 21th edition, 2005, Mack Publishing Company, Easton, Pa.

The subject compositions are administered in a cosmetically, dermatologically, pharmaceutically (including therapeutically, prophylactically and diagnostically) or dietary effective amount. The amount of the composition administered will typically be determined by a physician, in the light of the relevant circumstances, including the condition to be treated, the chosen route of administration, the age, weight, and response of the individual patient, the severity of the patient's symptoms, and the like.

The subject compositions may be administered to effect various forms of release, which include, without limitation, immediate release, extended release, controlled release, timed release, sustained release, delayed release, long acting, pulsatile delivery, etc., using well known procedures and techniques available to the ordinary skilled artisan. A description of representative sustained release materials can be found in the incorporated materials in Remington's Pharmaceutical Sciences.

The invention also relates to the use of an effective amount of the composition as defined above for the preparation of a cosmetic, pharmaceutical composition or dietary supplement for exerting antiradical and/or antiinflammatory activity.

Specifically, by virtue of their high antiradical activity the composition of the invention shows anti-inflammatory activity, particularly activity against the free radicals generated during a UV stress or the like induce the inflammation cascade.

The composition of the invention may be administered with beneficial effects to prevent, slow the progression of, or treat any disease associated with free radical damage, especially damage resulting in lipid peroxidation. This category includes CHD, rheumatism and inflammation, hepatitis, alcoholism, Alzheimer's disease, dementia, diabetes, multiple sclerosis, HIV and AIDS, collagen degradation, cataracts, macular degeneration, accelerated aging, neuropathies, myopathies, ischemia-reperfusion injury, haemorrhagic shock, gum disease, cold sores, psoriasis, eczema, seborrhea. The invention can also be applied to counteract the effects of formation of reactive oxygen species (ROS), whatever the cause, including that resulting from environmental pollution or irradiation, and from treatment with neoplastic drugs.

Other uses of the invention relates to biomolecules protection, particularly collagen, elastine, DNA and telomerase protection; anti-skin ageing; anti-wrinkles; to counteract the development of wrinkles, delay skin ageing, and improve the overall appearance of skin, eyes and hair; to avoiding or minimizing oxidation damage consequent upon physical activity, and stimulate cell skin renewal and repair.

As used herein, the following terms are used to describe the present invention. The definitions provided below, within context, may be used exclusively, or may be used to supplement definitions which are generally known to those of ordinary skill in the art.

### DEFINITIONS

The term "cosmetically acceptable excipient" means in the context of this invention any excipient that is cosmetically tolerated (most of the time meaning not being toxic) if used appropriately for a cosmetic treatment especially if used on or applied to humans and/or mammals.

The term "pharmaceutically acceptable excipient" means in the context of this invention any excipient that is pharmaceutically tolerated (most of the time meaning not being toxic) if used appropriately for a pharmaceutical treatment especially if used on or applied to humans and/or mammals.

Throughout the description and claims the word "comprise" and variations of the word, such as "comprising", are not intended to exclude other technical features, additives, components, or steps.

Additional objects, advantages and features of the invention will become apparent to those skilled in the art upon examination of the description or may be learned by practice of the invention. The following examples and drawings are provided by way of illustration.

### DETAILED DESCRIPTION OF PARTICULAR EMBODIMENTS

### Example 1. Protection of UV stress

HaCaT keratinocyte cells (Boukamp et al. J. Cell Biol. 1988.106: 761-771) were grown in Dulbecco's modified Eagle's medium (Sigma) supplemented with foetal bovine serum penicillin and streptomycin (Cambrex). For subculturing the cells were detached with tripsin/EDTA (Invitrogen) and were seeded at 1000 cels/well in 96 well microplates and incubated for 18 hours at 37°C and 5% CO₂.

After 24 hours the cells were incubated with fresh medium containing the test products (pycnogenol^{®} (P), gamma-tocotrienol (T) and niacinamide(N)) and mixtures (pycnogenol^{®} + gamma-tocotrienol (P+T), and pycnogenol^{®} + gamma-tocotrienol + niacinamide (P+T+N)). 24 hours later the medium was removed and cells were washed with phosphate buffered saline and exposed UVB irradiation with 50 mJ/cm² in phosphate buffered saline. The UV source used was a Bio Sun lamp (Vilber Lourmat)

After the UVB irradiation the cells were maintained in fresh culture medium for 24 hours. Viability was measured by WST-1 (Roche Applied Science) 24 hours after irradiation.

The results show a statistical difference increase in % viability only in the pycnogenol^{®} + gamma-tocotrienol + niacinamide (P+T+N) mixture treatment (increment 21.9%). Figure 1 shows the % viability of all treatments.

### Example 2. Proliferation study

HaCaT keratinocyte cells were grown in Dulbecco's modified Eagle's medium (Sigma) supplemented with foetal bovine serum penicillin and streptomycin (Cambrex). For subculturing the cells were detached with tripsin/EDTA (Invitrogen) and were seeded at 1000 cels/well in 96 well microplates and incubated for 18 hours at 37°C and 5% CO₂.

After 24 hours the cells were incubated with fresh medium containing the test products (pycnogenol^{®} (P), gamma-tocotrienol (T) and niacinamide(N)) and mixtures (pycnogenol^{®} + gamma-tocotrienol + niacinamide (P+T+N)). 24 hours later the medium was removed and cells were washed with phosphate buffered saline and maintained in fresh culture medium for 24 hours. Viability was measured by WST-1 (Roche Applied Science).

The results show a statistical difference increase in % viability with the pycnogenol^{®} + gamma-tocotrienol + niacinamide (P+T+N) mixture treatment. Figure 2 shows the % viability of all treatments.

### Example 3. Formulation

The following formulation examples illustrate representative cosmetic, dermatologic and pharmaceutical compositions of the invention.

The designation of the raw materials below is in accordance with the admitted International Nomenclature of Cosmetic Ingredients (INCI).

### DAY CREAM

| | |
|---|---|
| Butyrospermum parkii - shea butter | 4 g |
| Dimethicone | 3 g |
| Cetearyl alcohol | 2 g |
| Glycerin | 2 g |
| Glyceryl stearate S.E. | 2 g |
| Propylene Glycol | 2 g |
| Cetyl palmitate | 2 g |
| Ethylhexyl palmitate | 2 g |
| PEG-100 stearate | 1 g |
| Mannitol | 1 g |
| Hydrogenated lecithin | 1 g |
| Prunus Amigdalus Dulcis - Sweet almond oil | 1 g |
| Squalane | 0,75 g |
| Palmitic acid | 0,50 g |
| Carbomer | 0,40 g |
| Tromethamine | 0,40 g |
| Polysorbate 60 | 0,30 g |
| Gamma-tocotrienol | 0.1 g |
| Pinus pinaster bark extract | 0.1 g |
| Niacinamide | 0.1 g |
| Preservatives | q.s |
| Fragrance | q.s. |
| Water | q.s. 100 w/w |

### EYE CONTOUR CREAM

| | |
|---|---|
| Cetyl palmitate | 3 g |
| Isodecyl isononanoate | 2,5 g |
| Butyrospermum parkii - shea butter | 2. g |
| Myreth-3 myristate | 2 g |
| Glycerin | 3 g |
| Cetearyl alcohol | 2 g |
| Heliantthus annus - Sunflower seed oil | 1,50 g |
| Sorbitol | 1,50 g |
| Dimethicone | 1 g |
| Ethylhexyl palmitate | 1 g |
| Hydrogenated lecithin | 0,70 g |
| Propylene Glycol | 0,70 g |
| Acrylates - vinylisodecanoate crosspolymer | 0,40 g |
| Tromethamine | 0,50 g |
| Stearic acid | 0,50 g |
| Gamma-tocotrienol | 0.1 g |
| Pinus pinaster bark extract | 0.1 g |
| Niacinamide | 0.1 g |
| Preservatives | q.s. |
| Fragrance-Parfum | q.s. |
| Water | q.s. 100 w/w |

### BODY MILK.

| | |
|---|---|
| Cetearyl ethylhexanoate | 4 g |
| Butylene Glycol | 2 g |
| Glyceryl stearate SE | 2,50 g |
| Squalane | 2 g |
| PEG-100 Stearate | 2 g |
| Ethylhexyl palmitate | 1 g |
| Lecithin | 0,30 g |
| Glycerin | 2 g |
| Propylene Glycol | 1 g |
| Dimethicone | 0,75 g |
| Cetyl alcohol | 0,60 g |
| Tromethamine | 0,25 g |
| Carbomer | 0,20 g |
| Acrylates - vinylisodecanoate crosspolymer | 0,10 g |
| Gamma-tocotrienol | 0.1 g |
| Pinus pinaster bark extract | 0.1 g |
| Niacinamide | 0.1 g |
| Preservatives | q.s. |
| Fragrance | q.s. |
| Water | q.s. 100 w/w |

### MOUTHWASH

| | |
|---|---|
| Sorbitol | 5 g |
| Porpyleneglycol | 10 g |
| PEG-60 Hydrogenated castor oil | 2 g |
| Gamma-tocotrienol | 0.1 g |
| Pinus pinaster bark extract | 0.1 g |
| Niacinamide | 0.1 g |
| Fragrance | q.s. |
| Water | q.s.. 100 w/w |

## Claims

1. A composition comprising:
a) a source of proanthocyanidins which is a pine bark extract from *Pinus pinaster,*
b) gamma-tocotrienol; and
c) niacin; and
wherein the composition comprises:
i) between 0.01% and 2% by weight of proanthocyanidins;
ii) between 0.01% and 2% by weight of gamma-tocotrienol; and
iii) between 0.01 % and 2% by weight of niacin.

2. The compositions according to claim 1, wherein gamma-tocotrienol is provided in a Tocotrienol Rich Fraction with an amount of delta-tocols below 2% by weight.

3. The composition according to any of claims 1 to 2, wherein the niacin is niacinamide.

4. The composition according to any of claims 1 to 3, wherein the content of proanthocyanidins, gamma-tocotrienol and niacin in the composition is between 0.5 and 1 % by weight for each component.

5. A pharmaceutical or cosmetical composition or a dietary supplement comprising a therapeutical, cosmetical or dietary effective amount of the composition as defined in any of claims 1 to 4 and pharmaceutically, cosmetically or dietary acceptable excipients or carriers.

6. Composition according to any of claims 1 to 4 for use as a medicament.

7. Composition as defined in any of claims 1 to 4, for use in the prevention and/or treatment of a disease or a condition associated with the generation of free radicals selected from the group consisting of inflammation associated with the generation of free radicals generated during a UV stress, collagen degradation, cold-sores, psoriasis, eczema, and seborrhea.

8. Composition as defined in any of claims 1 to 4, for the prevention and/or treatment of skin ageing or wrinkles.

9. Composition as defined in any of the claims 1-4, which is formulated as a pharmaceutical composition, for use according to claim 7.

10. Composition as defined in any of the claims 1-4, which is formulated as a dietary or cosmetic composition, for use according to any of claims 7-8.

11. Composition for use according to any of claims 7-10, which is formulated for topical administration.

12. Composition for use according to claim 11 which is formulated for topical administration on the skin or the oral cavity.

## Patentansprüche

1. Eine Zusammensetzung umfassend:
a) eine Quelle von Proanthocyanidinen, welche ein Kiefernrindenextrakt aus *Pinus pinaster* ist,
b) Gamma-Tocotrienol; und
c) Niacin; und
wobei die Zusammensetzung folgendes umfasst:
i) zwischen 0,01 Gew.-% und 2 Gew.-% Proanthocyanidine
ii) zwischen 0,01 Gew.-% und 2 Gew.-% Gamma-Tocotrienol; und
iii) zwischen 0,01 Gew.-% und 2 Gew.-% Niacin.

2. Die Zusammensetzung nach Anspruch 1, wobei das Gamma-Tocotrienol in einer tocotrienolreicher Fraktion mit einem Anteil an Delta-Tocolen unter 2 Gew.-% bereitgestellt ist.

3. Die Zusammensetzung nach einem der Ansprüche 1 bis 2, wobei das Niacin Niacinamid ist.

4. Die Zusammensetzung nach einem der Ansprüche 1 bis 3, wobei der Anteil an Proanthocyanidinen, Gamma-Tocotrienol und Niacin in der Zusammensetzung bei zwischen 0,5 Gew.-% und 1 Gew.-% für jeden Bestandteil liegt.

5. Eine pharmazeutische oder kosmetische Zusammensetzung oder ein Nahrungsergänzungsmittel umfassend eine therapeutische, kosmetische oder diätetische wirksame Menge der Zusammensetzung wie in einem der Ansprüche 1 bis 4 definiert und pharmazeutisch, kosmetisch oder diätetisch akzeptable Hilfsstoffe oder Trägerstoffe.

6. Zusammensetzung nach einem der Ansprüche 1 bis 4 zur Verwendung als Arzneimittel.

7. Zusammensetzung wie in einem der Ansprüche 1 bis 4 definiert zur Verwendung bei der Vorbeugung und/oder bei der Behandlung einer Krankheit oder eines Zustands, die/der mit der Entstehung freier Radikale zusammenhängt, die/der ausgewählt ist aus der Gruppe bestehend aus Entzündung, die mit der Entstehung aufgrund UV-Belastung entstehender freier Radikale zusammenhängt, Kollagenabbau, Lippenherpes, Psoriasis, Ekzem, und Seborrhö.

8. Zusammensetzung wie in einem der Ansprüche 1 bis 4 definiert für die Vorbeugung und/oder für die Behandlung von Hautalterung oder von Hautfalten.

9. Zusammensetzung wie in einem der Ansprüche 1 bis 4 definiert, welche als eine pharmazeutische Zusammensetzung formuliert ist, zur Verwendung nach Anspruch 7.

10. Zusammensetzung wie in einem der Ansprüche 1 bis 4 definiert, welche als eine diätetische oder als eine pharmazeutische Zusammensetzung formuliert ist, zur Verwendung nach einem der Ansprüche 7-8.

11. Zusammensetzung zur Verwendung nach einem der Ansprüche 7-10, welche zur topischen Anwendung formuliert ist.

12. Zusammensetzung zur Verwendung nach Anspruch 11, welche zur topischen Anwendung auf die Haut oder in die Mundhöhle formuliert ist.

## Revendications

1. Une composition comprenant:
a) une source de proanthocyanidines qui est un extrait d'écorce de pin de *Pinus pinaster,*
b) gamma-tocotriénol; et
c) niacine; et
dans laquelle la composition comprend:
i) entre 0,01 % et 2% en poids de proanthocyanidines;
ii) entre 0,01 % et 2% en poids de gamma-tocotriénol; et
iii) entre 0,01% et 2% en poids de niacine;

2. La composition selon la revendication 1, dans laquelle le gamma-tocotriénol est fourni dans une fraction riche en tocotriénol avec une quantité de delta-tocols inférieure à 2% en poids.

3. La composition selon l'une quelconque des revendications 1 à 2, dans laquelle la niacine est la niacinamide.

4. La composition selon l'une quelconque des revendications 1 à 3, dans laquelle la teneur en proanthocyanidines, gamma-tocotriénol et niacine dans la composition est entre 0,5 et 1% en poids pour chaque constituant.

5. Une composition pharmaceutique ou cosmétique ou un complément alimentaire comprenant une quantité thérapeutique, cosmétique ou diététique efficace de la composition telle que définie dans l'une des revendications 1 à 4 et des excipients ou véhicules pharmaceutiquement, cosmétiquement ou diététiquement acceptables.

6. Composition selon l'une quelconque des revendications 1 à 4 pour son utilisation en tant que médicament.

7. Composition telle que définie dans l'une des revendications 1 à 4 pour son utilisation dans la prévention et/ou dans le traitement d'une maladie ou d'un état associé(e) à la génération de radicaux libres choisi(e) dans le groupe constitué des inflammations associées à la génération de radicaux libres générés pendant le stress UV, la dégradation du collagène, le feu sauvage, la psoriasis, l'eczéma, et la séborrhée.

8. Composition telle que définie dans l'une quelconque des revendications 1 à 4 pour le prévention et/ou le traitement du vieillissement de la peau ou des rides.

9. Composition telle que définie dans l'une quelconque des revendications 1 à 4, qui est formulée comme une composition pharmaceutique, pour son utilisation selon la revendication 7.

10. Composition telle que définie dans l'une quelconque des revendications 1 à 4, qui est formulée comme une composition diététique ou cosmétique, pour son utilisation selon l'une quelconque des revendications 7-8.

11. Composition pour son utilisation selon l'une quelconque des revendications 7 à 10, qui est formulée pour son administration topique.

12. Composition pour son utilisation selon la revendication 11, qui est formulé pour l'administration topique sur la peau ou dans la cavité orale.
